# EUROPEAN PATENT SPECIFICATION

(11) **EP 2 949 322 B1**
(45) Date of publication and mention of the grant of the patent: **25.04.2018**
(21) Application number: 15169362.9
(22) Date of filing: 27.05.2015
(51) Int. Cl.: A61K 9/24, A61K 31/4045

(54) **FORMULATION FOR ORAL ADMINISTRATION COMPRISING MELATONIN IN STABLE FORM AND METHOD OF PRODUCTION THEREOF**
FORMULIERUNG ZUR ORALEN VERABREICHUNG MIT MELATONIN IN STABILER FORM UND HERSTELLUNGSVERFAHREN DAFÜR
FORMULATION POUR ADMINISTRATION ORALE COMPRENANT DE LA MELATONINE EN FORME STABLE ET SON PROCEDE DE PRODUCTION

(30) Priority: 28.05.2014 IT FI20140128
(43) Date of publication of application: 02.12.2015
(73) Proprietor: Valpharma S.p.A., 47899 Serravalle (SM)
(72) Inventor: VALDUCCI, Roberto, 47039 Savignano sul Rubicone (IT); AVANESSIAN, Serozh, 47827 Villa Verucchio (IT)
(74) Representative: Valenza, Silvia

(56) References cited:
- WO-A1-2012/013595
- WO-A2-2011/107750
- KUMAR A ET AL: "MODIFIED RELEASE BI-LAYERED TABLET OF MELATONIN USING BETA-CYCLODEXTRIN", DIE PHARMAZIE, GOVI VERLAG PHARMAZEUTISCHER VERLAG GMBH, ESCHBORN, DE, vol. 58, no. 9, 1 September 2003 (2003-09-01), pages 642-644, XP001170932, ISSN: 0031-7144
- RINA CHOKSHI ET AL: "Hot-Melt Extrusion Technique: A Review", IRANIAN JOURNAL OF PHARMACEUTICAL RESEARCH, vol. 3, no. 1, 1 January 2004 (2004-01-01) , pages 3-16, XP055054771,
- Paul J Sheskey, Walter G Cook and Colin G Cable: "Glyceryl Behenate", Medicines Complete Handbook of Pharmaceutical Excipients, 2014, XP002733302, Retrieved from the Internet: URL:https://www.medicinescomplete.com/mc/e xcipients/current/1001938956.htm?q=behenat e&t=search&ss=text&p=2#_hit

## Description

### FIELD OF THE INVENTION

The present invention relates to the field of pharmaceutical formulations, and in particular relates to a formulation comprising melatonin and method of production thereof.

### PRIOR ART

Melatonin is a hormone which is secreted by the pineal gland, a small gland located near the centre of the brain. Its chemical formula is N-acetyl-5-methoxytryptamine which is a derivative of the amino acids tryptophan and serotonin. The pineal gland secretes melatonin to control the circadian rhythms that affect biochemical, physical and behavioural processes in the body. Circadian rhythms are endogenous processes and are adjusted by external stimuli, such as daylight. It is known that plasma concentrations of melatonin are low during the day and rise during the early evening, maintaining a constant concentration of 25-120 pg/mL during the night, until the following morning.

Exogenous or synthetic melatonin is used clinically to treat melatonin deficiencies in the elderly and to regulate sleep disorders, jet lag and season disorders. Some studies suggest that melatonin may also be effective in the treatment of breast cancer, fibrocystic breast conditions and colon cancer. It is also used to enhance immune responses, reduce stress, improve sleep disorders in patients with Alzheimer's disease and oxidative stress.

As is known, insomnia is a widespread illness and melatonin remains the best medication for treating and improving this problem.

Melatonin, a physiological hormone in mammals, follows circadian rhythms and in a healthy patient increases in the evening, reaching its maximum levels during the night and then decreases in the morning upon waking. The drug, which simply mimics the effects and physiological levels of melatonin in the blood, thereby offsetting pathological deficiencies, must achieve sufficient haematic levels to carry out their function in the first few minutes following administration, so as to quickly put the patient to sleep, but must also maintain a sufficient concentration in the blood throughout the sleep period to avoid micro-awakenings and let the person rest for at least 8 hours.

Existing literature includes various formulations containing melatonin which all aim to offset endogenous deficiencies of this hormone.

Patent US2004/0043065A1 describes a tablet containing controlled release melatonin, which consists of an interior slow release nucleus and an exterior immediate release "cortex", both containing melatonin in equal or different doses. The delayed release internal nucleus is responsible for the extended release of the active ingredient and, to solve the issues of initial deficiencies, the nucleus is coated with a membrane containing an amount of melatonin which provides an immediate release as soon as the tablet reaches the stomach and the active ingredient is absorbed. It is immediately apparent from this production method that there are difficulties in being able to ensure a constant quantity of melatonin between the various tablets and product batches, considering that the process of applying the active ingredient as a solution to the tablets is an uncontrollable process and subject to massive and irregular processing losses. The fact that melatonin becomes unstable upon contact with water and produces impurities during stability studies cannot be overlooked.

Patent US2012/0195968A1 describes a controlled-release formulation containing melatonin. Based on the fact that melatonin has greater solubility in an acidic environment, the formulation provides for combining the active ingredient with a polymer matrix which can maintain an acidic pH within the pharmaceutical form even in the presence of various external pH environments. In practice, it aims to maintain within the tablet an acidic environment, which is stable and adapted to the bioavailability of melatonin, in the gastrointestinal tract, where otherwise it would only have high solubility in the stomach. To do this, organic acids (e.g. citric acid) or surfactants (e.g. PEG) are added to the formulation.

The tablet is generally formed from an acidified polymer matrix. Sometimes such a tablet may be covered with a coating which insulates the nucleus from the external environment, and the acidifying agent is added at this level; a further coating may also be present which may or may not be functional, and may also contain a portion of the active ingredient so as to have an immediate release part.

Further relevant prior art includes KUMAR A. ET AL. "MODIFIED RELEASE BILAYERED TABLET OF MELATONIN USING BETA-CYCLODEXTRIN" DIE PHARMAZIE, GOVI VERLAG PHARMAZEUTICHER VRLAG GMBH, ESCHBORN, DE, vol. 58, no. 9, 1 September 2003 which discloses a bi-layer tablet comprising melatonin, wherein the tablet composition comprises a fast release layer and a controlled release layer where both layer comprise melatonin.

The present invention aims to provide a formulation, which is at least an alternative to those mentioned in the prior art, wherein part of the melatonin is released immediately, while the remaining part is released gradually over 8-10 hours.

### DEFINITIONS AND ABBREVIATIONS

API: Active Pharmaceutical Ingredient

### SUMMARY OF THE INVENTION

The present formulation solves the problems above with an pharmaceutical formulation to be orally administered, that is a pharmaceutical composition in the form of a bilayer tablet comprising melatonin as the active pharmaceutical ingredient (API), said tablet comprising:
a first layer consisting of a first granulate containing 10%-30% API, a glyceric derivative of saturated fatty acids having C20-C24 chains and other excipients which allow an immediate release of the API;
a second layer consisting of a second granulate containing 70-90% API, a glyceric derivative of saturated fatty acids having C20-C24 chains and other excipients which allow an extended release of the API;
each granulate obtained by hot granulation at a temperature from 77 to 83 °C for 8-12 minutes;
wherein said % are % with respect to the total weight of the API in the bilayer tablet.

It is clear that, according to this formulation approach, the ratio between the quantity of immediate release and extended release melatonin can be varied according to requirements. In this way various types of melatonin deficiencies, from the mildest to the most severe, can be treated. In the case of severe deficiencies greater immediate release is required and so the percentage of melatonin in the outer layer is increased; for mild deficiencies the instant portion can be much less. Moreover, a tablet according to the present invention may contain melatonin in a quantity ranging from 1 mg to 3 mg.

The present invention is characterised by the following advantages:
- In the formulation according to the invention, melatonin is present in a form which is stable over time; this stability is provided by hot granulation reaching a temperature of around 80 °C which is then maintained for around 10 minutes. In this way, the active ingredient, upon contact with the stabilising excipient, which at this temperature is present in its malleable and "softened" form, takes on a form which renders it stable over time, avoiding the development of impurities and related substances during the performance of in-vitro stability studies. In addition, granulation by heating and without moisture avoids the active ingredient coming into contact with water which would make it unstable.
- Hot granulation not only prevents the matrix effect, as the molten wax mixes better with the melatonin but avoids demixing following direct compression. Being the melatonin raw material finely granulated and having excipients larger particles sizes, direct compression of a wet granulate could lead to demixing.

The formulation according to the invention, being composed of two different release layers, solves the problem of melatonin deficiencies in the blood in the first few minutes following administration. In fact, one layer has an immediate release of 10%-30% of active ingredient, followed by a slow and extended release of the remaining quantity from the controlled layer.

The formulation in question inserts the instant portion of melatonin into a layer of a bilayer tablet and does not apply it to the surface of a controlled nucleus like other formulations mentioned in the prior art. As the immediate-release layer is very low dosage, since it is one layer and not applied to the surface of a controlled nucleus, undesirable differences in content uniformity between the various tablets and product batches are avoided. This is a common problem when active ingredients are applied in a solution, because this process is highly variable and subject to massive, uneven and unpredictable losses. Another problem which is avoided by the formulation according to the invention, through the bilayer approach, is that the melatonin is not placed in a solution, a process which usually leads to the development of impurities during stability studies.

### DETAILED DESCRIPTION OF THE INVENTION

Preferably, the glyceric derivative of saturated fatty acids having C20-C24 chains is selected from the group consisting of arachidic acid, behenic acid, and lignoceric acid; more preferably glyceryl behenate.

Preferably, the immediate release layer comprises the glyceric derivative of saturated fatty acids having C20-C24 chains, used as a stabilizer, in a percentage from 0.5% to 4%, more preferably 1.5% to 3% with respect to the total weight of the layer.

Preferably, the extended release layer comprises the glyceric derivative of saturated fatty acids having C20-C24 chains, used as a stabilizer while being responsible for the extended release, in a percentage from 4% to 30%, preferably from 5% to 25%, more preferably from 10% to 20%, with respect to the total weight of the layer.

Preferably, the immediate release layer comprises, as other excipients, a diluent, at least one disintegrant, a lubricant and a glidant.

Preferably, the extended release layer comprises, as other excipients, a diluent, a water-soluble excipient, a lubricant and a glidant.

Preferably, the diluent is microcrystalline cellulose.

Preferably, the disintegrant is selected from Carboxymethyl-starch, Croscarmellose sodium and mixtures thereof.

Preferably, the lubricant is anhydrous colloidal silica or sodium stearyl fumarate.

Preferably, the glidant is magnesium stearate or talc.

Preferably, the water-soluble excipient, which acts as canalization within the matrix, is maltodextrin, mannitol or lactose.

Preferably, the diluent is present in the immediate release layer in a percentage from 85% to 95%, preferably from 87% to 93%, with respect to the total weight of the layer.

Preferably, the diluent is present in the extended release layer in a percentage from 10% to 50%, preferably from 20% to 40%, with respect to the total weight of the layer.

Preferably, the glidants and lubricants are present in a percentage from 0.25% to 2%, preferably from 0.5% to 1 %, with respect to the total weight of the layer.

Preferably, the entire disintegrants are present in a percentage from 1% to 10%, preferably from 2% to 8%, with respect to the total weight of the layer.

Preferably, the soluble excipient is present in a percentage from 20% to 80%, preferably from 30% to 70%, more preferably from 40% to 60%, with respect to the total weight of the layer.

The present invention also relates to a method of producing a bilayer tablet according to the invention, said method comprising hot granulation and subsequent compression of each layer.

Preferably, for each layer, granulation is carried out by mixing the components of the layer, excluding lubricant and glidant, and the mixture is heated under stirring until reaching a temperature of 80 °C ± 3 °C, and stirred again at constant temperature for 8-12 minutes, thus obtaining a granulate which is then sieved in a vibrating screen while it is still hot;
then, after cooling, each granulate is mixed with the compression excipients, lubricant and glidant, and compressed in a bilayer tableting machine.

By granulating in this way, the active ingredient, upon contact with the stabilising excipient, which at this temperature is present in its malleable and "softened" form, takes on a form which renders it stable over time, avoiding the development of impurities and related substances during the performance of in-vitro stability studies.

The increased stability is undoubtedly provided by the fact that the melatonin never comes into contact with solvents, nor granulation nor solution/suspension, which are also responsible for the development of impurities in in-vitro stability studies. The melatonin is processed "dry" throughout the productive cycle, avoiding mechanical and chemical stresses which would alter the structure and consequently the pharmacological action.

Specifically in the controlled layer, it is evident that it is precisely this step in the production which leads to achieving the desired transfer. In fact, by inserting a sufficient quality of stabilising excipient and delaying agent (waxy in nature), and heating the product, the mixture is better united with the active ingredient, thereby increasing the effect of the controlled matrix, which is otherwise insufficient if produced by direct compression. It is noted that this effect only occurs upon reaching a temperature of 80 °C ± 3 °C for 8-12 minutes.

The other advantage of granulation is certainly the increased content uniformity of the active ingredient in the tablets. The melatonin present on the market is very finely granulated. Upon contact with the other components in the layers, having different and larger particle sizes, and with wet granulation, demixing of the mix would occur with greater losses of the finer active ingredient compared with those of the excipients. With hot granulation instead, the melatonin is incorporated into the granules, thereby avoiding this problem.

The present invention will be better understood in the light of the following embodiments.

### EXPERIMENTS

### EXAMPLE 1:

### Dosage of 2 mg (10% immediate release and 90% extended release)

### -Production - 1 st layer

| **COMPONENTS** | **QUANTITY (g)** | **PERCENTAGE (%)** | **FUNCTION** |
|---|---|---|---|
| MELATONIN | 4 | 0.4 | API |
| MICR. CELLULOSE | 896 | 89.6 | DILUENT |
| GLYCERYL DIHEBENATE | 20 | 2 | STABILIZER |
| CARBOXYMETHYL STARCH | 40 | 4 | DISINTEGRANT |
| CROSCARMELLOSE SODIUM | 30 | 3 | DISINTEGRANT |
| MAGNESIUM STEARATE | 20 | 2 | GLIDANT |
| ANHYDROUS COLLOIDAL SILICA | 10 | 1 | LUBRICANT |
| Total | 1000 | 100% | |

The first five components are mixed and then heated until reaching a temperature of 80°C, stirring continuously at a constant temperature for 10 minutes. After sifting and cooling the granulate, it is mixed with glidants and lubricants.

### -Production - 2nd layer

| **COMPONENTS** | **QUANTITY (g)** | **PERCENTAGE (%)** | **FUNCTION** |
|---|---|---|---|
| MELATONIN | 12 | 1.2 | API |
| GLYCERYL DIHEBENATE | 150 | 15 | STABILIZER and DELAYING AGENT |
| MALTODEXTRIN | 526 | 52.6 | SOLUBLE EXCIPIENT |
| MICR. CELLULOSE | 300 | 30 | DILUENT |
| MAGNESIUM STEARATE | 6 | 0.6 | GLIDANT |
| ANHYDROUS COLLOIDAL SILICA | 6 | 0.6 | LUBRICANT |
| Total | 1000 | 100% | |

The first four components are mixed and then heated until reaching a temperature of 80°C, stirring continuously at a constant temperature for 10 minutes. After sifting and cooling the granulate, it is mixed with glidants and lubricants.

### -Compression

The above-mentioned granulate is compressed using a bilayer tableting machine equipped with ∅8mm round punches, at a dose of 2 mg of melatonin, with a ratio of active ingredient between the immediate and extended layers of 10% - 90%.

### -In-vitro analysis:

The tablets are analysed using paddles (50 rpm) and a sinker, in pH 6.8 phosphate buffer /1000 ml, achieving the following release profile:

| **TIME** | **RELEASE PERCENTAGE** | **RELEASE SPECIFICATIONS** |
|---|---|---|
| 5 mins | 16% | > 10% |
| 15 mins | 26% | > 20% |
| 30 mins | 34% | 25% - 40% |
| 1 h | 45% | 40% - 50% |
| 2 hrs | 59% | 50% - 60% |
| 4 hrs | 76% | 70% - 80% |
| 6 hrs | 87% | 80% - 90% |
| 8 hrs | 94% | > 85% |

### EXAMPLE 2:

### Dosage of 1 mg (20% immediate release and 80% extended release)

### -Production - 1 st layer

| **COMPONENTS** | **QUANTITY (g)** | **PERCENTAGE (%)** | **FUNCTION** |
|---|---|---|---|
| MELATONIN | 2 | 0.2 | API |
| MICR. CELLULOSE | 917 | 91.7 | DILUENT |
| GLYCERYL DIHEBENATE | 15 | 1.5 | STABILIZER |
| CARBOXYMETHYL STARCH | 15 | 1.5 | DISINTEGRANT |
| CROSCARMELLOSE SODIUM | 20 | 2 | DISINTEGRANT |
| MAGNESIUM STEARATE | 20 | 2 | GLIDANT |
| ANHYDROUS COLLOIDAL SILICA | 10 | 1 | LUBRICANT |
| Total | 1000 | 100% | |

The first five components are mixed and then heated until reaching a temperature of 80°C, stirring continuously at a constant temperature for 10 minutes. After sifting and cooling the granulate, it is mixed with glidants and lubricants.

### -Production - 2nd layer

| **COMPONENTS** | **QUANTITY (g)** | **PERCENTAGE (%)** | **FUNCTION** |
|---|---|---|---|
| MELATONIN | 6 | 0.6 | API |
| GLYCERYL DIHEBENATE | 180 | 18 | STABILIZER and DELAYING AGENT |
| MALTODEXTRIN | 480 | 48 | SOLUBLE EXCIPIENT |
| MICR. CELLULOSE | 325 | 32.5 | DILUENT |
| MAGNESIUM STEARATE | 6 | 0.6 | GLIDANT |
| ANHYDROUS COLLOIDAL SILICA | 3 | 0.3 | LUBRICANT |
| Total | 1000 | 100 % | |

The first four components are mixed and then heated until reaching a temperature of 80°C, stirring continuously at a constant temperature for 10 minutes. After sifting and cooling the granulate, it is mixed with glidants and lubricants.

### -Compression

The above-mentioned granulate is compressed using a bilayer tableting machine equipped with ∅8mm round punches, at a dose of 1 mg of melatonin, with a ratio of active ingredient between the instant and extended layers of 20% - 80%.

### In-vitro analysis:

The tablets are analysed using paddles (50 rpm) and a sinker, in pH 6.8 phosphate buffer /1000 ml, achieving the following release profile:

| **TIME** | **RELEASE PERCENTAGE** | **RELEASE SPECIFICATIONS** |
|---|---|---|
| 5 mins | 20% | > 10% |
| 15 mins | 29% | > 20% |
| 30 mins | 38% | 25% - 40% |
| 1 h | 49% | 40% - 50% |
| 2 hrs | 59% | 50% - 60% |
| 4 hrs | 78% | 70% - 80% |
| 6 hrs | 88% | 80% - 90% |
| 8 hrs | 98% | > 85% |

### EXAMPLE 3:

### Dosage of 3 mg (15% immediate release and 85% extended release)

### -Production - 1 st layer

| **COMPONENTS** | **QUANTITY (g)** | **PERCENTAGE (%)** | **FUNCTION** |
|---|---|---|---|
| MELATONIN | 6 | 0.6 | API |
| MICR. CELLULOSE | 924 | 92.4 | DILUENT |
| GLYCERYL DIHEBENATE | 30 | 3 | STABILIZER |
| CARBOXYMETHYL STARCH | 10 | 1 | DISINTEGRANT |
| CROSCARMELLOSE SODIUM | 10 | 1 | DISINTEGRANT |
| MAGNESIUM STEARATE | 10 | 1 | GLIDANT |
| ANHYDROUS COLLOIDAL SILICA | 10 | 1 | LUBRICANT |
| Total | 1000 | 100 % | |

The first five components are mixed and then heated until reaching a temperature of 80°C, stirring continuously at a constant temperature for 10 minutes. After sifting and cooling the granulate, it is mixed with glidants and lubricants.

### -Production - 2nd layer

| **COMPONENTS** | **QUANTITY (g)** | **PERCENTAGE (%)** | **FUNCTION** |
|---|---|---|---|
| MELATONIN | 18 | 1.8 | API |
| GLYCERYL DIHEBENATE | 120 | 12 | STABILIZER and DELAYING AGENT |
| MALTODEXTRIN | 580 | 58 | SOLUBLE EXCIPIENT |
| MICR. CELLULOSE | 270 | 27 | DILUENT |
| MAGNESIUM STEARATE | 6 | 0.6 | GLIDANT |
| ANHYDROUS COLLOIDAL SILICA | 6 | 0.3 | LUBRICANT |
| Total | 1000 | 100% | |

The first four components are mixed and then heated until reaching a temperature of 80°C, stirring continuously at a constant temperature for 10 minutes. After sifting and cooling the granulate, it is mixed with glidants and lubricants.

### -Compression

The above-mentioned granulate is compressed using a bilayer tableting machine equipped with ∅8mm round punches, at a dose of 3 mg of melatonin, with a ratio of active ingredient between the instant and extended layers of 15% - 85%.

### -In-vitro analysis:

The tablets are analysed using paddles (50 rpm) and a sinker, in pH 6.8 phosphate buffer /1000 ml, achieving the following release profile:

| **TIME** | **RELEASE PERCENTAGE** | **RELEASE SPECIFICATIONS** |
|---|---|---|
| 5 mins | 18% | > 10% |
| 15 mins | 27% | > 20% |
| 30 mins | 35% | 25% - 40% |
| 1 h | 47% | 40% - 50% |
| 2 hrs | 57% | 50% - 60% |
| 4 hrs | 77% | 70% - 80% |
| 6 hrs | 85% | 80% - 90% |
| 8 hrs | 96% | > 85% |

### Example 4 - Stability studies

Stability studies of the tablets according to the present invention are set up, in a climatic chamber with temperatures of 25 °C and relative humidity of 60% to verify the development of impurities. After three to six months the following results are obtained and are compared with the stability results of a formulation obtained according to traditional composition and production methods (i.e. CIRCADIN® 2 mg):

### -analysis at 3 months (25 °C - 60% RH):

| **IMPURITIES** | **LIMITS** | **PATENT FORMULATION** | **TRADITIONAL FORMULATION** |
|---|---|---|---|
| ***Known*** | 0.5% | 0.2% | 0.4% |
| ***Unknown*** | 1% | 0.4% | 1.2% |
| ***Totals*** | 1.5% | 0.6% | 1.6% |

### -analysis at 6 months (25 °C - 60% RH):

| **IMPURITIES** | **LIMITS** | **PATENT FORMULATION** | **TRADITIONAL FORMULATION** |
|---|---|---|---|
| ***Known*** | 0.5% | 0.3% | 0.6% |
| ***Unknown*** | 1% | 0.6% | 1.2% |
| ***Totals*** | 1.5% | 0.9% | 1.9% |

## Claims

1. A pharmaceutical composition in the form of a bilayer tablet comprising melatonin as active pharmaceutical ingredient (API), said tablet comprising:
a first layer consisting of a first granulate containing 10-30% API, a glyceric derivative of saturated fatty acids having C20-C24 chains and other excipients which allow an immediate release of the API;
a second layer consisting of a second granulate containing 70-90% API, a glyceric derivative of saturated fatty acids having C20-C24 chains and other excipients which allow an extended release of the API;
each granulate obtained by hot granulation at a temperature from 77 to 83 °C for 8-12 minutes;
wherein said % are wt% with respect to the total weight of the API in the bilayer tablet;
wherein the glyceric derivative of saturated fatty acids having C20-C24 chains is selected from the group consisting of arachidic acid, behenic acid, and lignoceric acid;
wherein the immediate release layer comprises the glyceric derivative of saturated fatty acids having C20-C24 chains, used as a stabilizer, in a percentage from 0.5wt% to 4wt% with respect to the total weight of the layer;
wherein the extended release layer comprises the glyceric derivative of saturated fatty acids having C20-C24 chains, used as a stabilizer while being responsible for the extended release, in a percentage from 4wt% to 30wt% with respect to the total weight of the layer.

2. A composition according to any one of the preceding claims, wherein the immediate release layer comprises, as other excipients, a diluent, at least one disintegrant, a lubricant and a glidant.

3. A composition according to any one of the preceding claims, wherein the extended release layer comprises, as other excipients, a diluent, a water-soluble excipient, a lubricant and a glidant.

4. A composition according to any one of the preceding claims, wherein the diluent is microcrystalline cellulose.

5. A composition according to any one of the preceding claims, wherein the disintegrant is selected from Carboxymethyl-starch, Croscarmellose sodium and mixtures thereof.

6. A composition according to any one of the preceding claims, wherein the lubricant is anhydrous colloidal silica or sodium stearyl fumarate and the glidant is magnesium stearate or talc.

7. A composition according to any one of the preceding claims, wherein the water-soluble excipient, which acts as canalization within the matrix, is maltodextrin, mannitol or lactose.

8. A method of producing a bilayer tablet according to any one of the preceding claims, said method comprising hot granulation and subsequent compression of each layer.

9. A method according to claim 8, wherein
for each layer, granulation is carried out by mixing the components of the layer, excluding lubricant and glidant, and the mixture is heated under stirring until reaching a temperature of 80 °C ± 3 °C, and stirred again at constant temperature for 8-12 minutes, thus obtaining a granulate which is then sieved in a vibrating screen while it is still hot;
then, after cooling, each granulate is mixed with the compression excipients, lubricant and glidant, and compressed in a bilayer tableting machine.

## Patentansprüche

1. Pharmazeutische Zusammensetzung in Form einer Zweischichttablette, die Melatonin als Wirkstoff (API) umfasst, wobei die Tablette umfasst:
ein erste Schicht, die aus einem ersten Granulat besteht, das 10-30% API, ein Glyzerinderivat von gesättigten Fettsäuren mit C20-C24-Ketten und weitere Hilfsstoffe, die eine sofortige Freigabe des API gestatten, enthält;
eine zweite Schicht, die aus einem zweiten Granulat besteht, das 70-90% API, ein Glyzerinderivat von gesättigten Fettsäuren mit C20-C24-Ketten und weitere Hilfsstoffe, die eine verlängerte Freigabe des API gestatten, enthält;
wobei jedes Granulat durch Heißgranulierung bei einer Temperatur von 77-83 °C für 8-12 Minuten erhalten wird;
wobei die % Gewichts-% mit Bezug auf das Gesamtgewicht des API in der Zweischichttablette sind;
wobei das Glyzerinderivat von gesättigten Fettsäuren mit C20-C24-Ketten aus der Gruppe ausgewählt ist, die aus Arachinsäure, Behensäure und Lignocerinsäure besteht;
wobei die Schicht mit sofortiger Freigabe das Glyzerinderivat von gesättigten Fettsäuren mit C20-C24-Ketten, das als Stabilisator verwendet wird, in einem Prozentsatz von 0,5 Gewichts-% bis 4 Gewichts-% mit Bezug auf das Gesamtgewicht der Schicht umfasst;
wobei die Schicht mit verlängerter Freigabe das Glyzerinderivat von gesättigten Fettsäuren mit C20-C24-Ketten, das als Stabilisator verwendet wird, während es für die verlängerte Freigabe verantwortlich ist, in einem Prozentsatz von 4 Gewichts-% bis 30 Gewichts-% mit Bezug auf das Gesamtgewicht der Schicht umfasst.

2. Zusammensetzung nach Anspruch 1, wobei die Schicht mit sofortiger Freigabe als weitere Hilfsstoffe ein Streckmittel, zumindest ein Desintegrationsmittel, ein Schmiermittel und ein Gleitmittel umfasst.

3. Zusammensetzung nach einem der vorhergehenden Ansprüche, wobei die Schicht mit verlängerter Freigabe als weitere Hilfsstoffe ein Streckmittel, einen wasserlöslichen Hilfsstoff, ein Schmiermittel und ein Gleitmittel umfasst.

4. Zusammensetzung nach einem der vorhergehenden Ansprüche, wobei das Streckmittel mikrokristalline Zellulose ist.

5. Zusammensetzung nach einem der vorhergehenden Ansprüche, wobei das Desintegrationsmittel aus Carboxymethylstärke, Croscarmellosenatrium und deren Mischungen ausgewählt ist.

6. Zusammensetzung nach einem der vorhergehenden Ansprüche, wobei das Schmiermittel wasserfreies, kolloidales Silica oder Natriumstearylfumarat ist, und das Gleitmittel Magnesiumstearat oder Talk ist.

7. Zusammensetzung nach einem der vorhergehenden Ansprüche, wobei der wasserlösliche Hilfsstoff, der als Kanalisation innerhalb der Matrix wirkt, Maltodextrin, Mannitol oder Lactose ist.

8. Verfahren zum Herstellen einer Zweitschichttablette nach einem der vorhergehenden Ansprüche, wobei das Verfahren Heißgranulierung und nachfolgend Komprimierung jeder Schicht umfasst.

9. Verfahren nach Anspruch 8, wobei
für jede Schicht die Granulierung durch Mischen der Komponenten der Schicht, unter Ausschluss des Schmiermittels und des Gleitmittels, ausgeführt wird, und die Mischung unter Rühren erwärmt wird, bis eine Temperatur von 80 °C ± 3 °C erreicht wird, und wieder bei konstanter Temperatur für 8-12 Minuten gerührt wird, wodurch ein Granulat erhalten wird, das dann in einem Schwingsieb gesiebt wird, während es noch heiß ist; und
dann, nach dem Abkühlen, jedes Granulat mit den Komprimierungshilfsstoffen, dem Schmiermittel und dem Gleitmittel gemischt wird und in einer Zweischicht-Tablettiermaschine komprimiert wird.

## Revendications

1. Composition pharmaceutique sous la forme d'un comprimé bicouche comprenant de la mélatonine en tant qu'ingrédient pharmaceutique actif (API), ledit comprimé comprenant :
une première couche constituée d'un premier granulé contenant 10 à 30 % d'API, d'un dérivé glycérique d'acides gras saturés ayant des chaînes C20-C24 et d'autres excipients qui permettent une libération immédiate de l'API ;
une seconde couche constituée d'un second granulé contenant 70 à 90 % d'API, d'un dérivé glycérique d'acides gras saturés ayant des chaînes C20-C24 et d'autres excipients qui permettent une libération prolongée de l'API ;
chaque granulé étant obtenu par granulation à chaud à une température de 77 à 83 °C pendant 8 à 12 minutes ;
dans lequel lesdit % sont des % en poids par rapport au poids total de l'API dans le comprimé bicouche ;
dans lequel le dérivé glycérique d'acides gras saturés ayant des chaînes C20-C24 est choisi parmi le groupe consistitué de l'acide arachidique, l'acide béhénique et l'acide lignocérique,
dans lequel la couche à libération immédiate comprend le dérivé glycérique d'acides gras saturés ayant des chaînes C20-C24, utilisé comme stabilisant, en pourcentage de 0,5 % en poids à 4 % en poids par rapport au poids total de la couche ;
dans lequel la couche à libération prolongée comprend le dérivé glycérique d'acides gras saturés ayant des chaînes C20-024, utilisé comme stabilisant tout en étant responsable de la libération prolongée, en pourcentage de 4 % en poids à 30 % en poids par rapport au poids total de la couche.

2. Composition selon l'une quelconque des revendications précédentes, dans laquelle la couche à libération immédiate comprend, comme autres excipients, un diluant, au moins un désintégrant, un lubrifiant et un agent de glissement.

3. Composition selon l'une quelconque des revendications précédentes, dans laquelle la couche à libération prolongée comprend, comme autres excipients, un diluant, un excipient hydrosoluble, un lubrifiant et un agent de glissement.

4. Composition selon l'une quelconque des revendications précédentes, dans laquelle le diluant est une cellulose microcristalline.

5. Composition selon l'une quelconque des revendications précédentes, dans laquelle le désintégrant est choisi parmi Carboxymethyl-amidon, Croscarmellose sodium et des mélanges de ceux-ci.

6. Composition selon l'une quelconque des revendications précédentes, dans laquelle le lubrifiant est de la silice colloïdale anhydre ou du stéaryle fumarate de sodium et l'agent de glissement est du stéarate de magnésium ou du talc.

7. Composition selon l'une quelconque des revendications précédentes, dans laquelle l'excipient hydrosoluble, qui agit comme une canalisation dans la matrice, est de la maltodextrine, du mannitol ou du lactose.

8. Procédé de production d'un comprimé bicouche selon l'une quelconque des revendications précédentes, ledit procédé comprenant une granulation à chaud et une compression ultérieure de chaque couche.

9. Procédé selon la revendication 8, dans lequel
pour chaque couche, la granulation s'effectue en mélangeant les composants de la couche, à l'exception du lubrifiant et de l'agent de glissement, et le mélange est chauffé tout en remuant jusqu'à atteindre une température de 80 °C±3 °C, et remué à nouveau à température constante pendant 8 à 12 minutes, obtenant ainsi un granulé qui est ensuite tamisé dans un écran vibrant alors qu'il est encore chaud ;
ensuite, après refroidissement, chaque granulé est mélangé avec les excipients de compression, le lubrifiant et l'agent de glissement, et comprimé dans une machine de formation de comprimé bicouche.
